# EUROPEAN PATENT APPLICATION

(11) **EP 2 749 320 A1**
(43) Date of publication of application: **02.07.2014**
(21) Application number: 11779082.4
(22) Date of filing: 31.08.2011
(51) Int. Cl.: A61Q 1/12, A61K 8/02, A61K 8/25, A61K 8/29, A61K 8/19, A61K 8/73, A61K 8/97, A61K 36/889

(54) **COSMETIC COMPOSITION INTENDED FOR MAKING UP THE SKIN, COSMETIC PRODUCT AND COSMETIC USE OF BABASSU POLYSACCHARIDES**

(71) Applicant: Natura Cosméticos S.A., 06882-700 Itapecerica da Serra SP (BR)
(72) Inventor: DEL REY CASTRIOTTO, Alciona, 05709-020 Sâo Paulo -SP (BR); DE SOUXA FERREIRA, Cinthia Fernanda, 06036-048 Osasco - SP (BR); GANDINI ANDRÉO, Luciana, 05025-010 Sâo Paulo - SP (BR); FARIA, Luciana, 06600-100 Jandira (BR); ARAUJO RODRIGUES MACHIUTTI, Pâmela, 09241-060 Santo André - Sp (BR); ESTEVES SOARES, Simone, 13214-584 Jundiaí -SP (BR)
(74) Representative: Pearson, Samuel John
(86) International application number: PCT/BR2011/000307
(87) International publication number: WO 2013/029126

(57) **Abstract**

The present invention relates to a cosmetic composition intended for making up the skin (face and body) comprising: (a) a sensory modifying system comprising at least one babassu polysaccharide; (b) at least one treated pigment; and (c) at least one light diffuser. Said cosmetic composition can be used for uniformly making up the skin, and when applied completely covers the region of the skin, adheres well, minimizes excessive shine, results in a smooth, satiny and velvety application to the skin, among other desired advantages for such a product. The present invention also relates to cosmetic products comprising said cosmetic composition. The present invention also relates to the cosmetic use of babassu polysaccharide as a sensory modifier.

## Description

### FIELD OF THE INVENTION

This invention addresses a cosmetic composition whose main application is for making up the skin. Furthermore, this invention refers to the cosmetic products encompassed by said cosmetic composition. These products are also preferably intended for making up the skin.

### BACKGROUND OF THE INVENTION

Make-up normally involves the use of colored materials that are placed on the body or face.

Some make-up compositions are presented in the form of loose or pressed powder. These compositions generally have high powder contents. Depending on the type of powder used, the properties of the cosmetic composition may vary, and may even be harmful to the skin, leaving it feeling dry and rough. Some make-up products in the form of powders leave the skin looking dull, and may even highlight irregularities on the skin surface, characteristic of micro-textures, wrinkles and fine lines.

In order to resolve these inconvenient aspects, many products and technologies have already developed, some of which are mentioned below:

Document WO 2007/07292 describes a cosmetic process and a make-up kit encompassing babassu oil and pigments based on iron oxide for the production of pressed or loose powder bases, concealers and eye shadows. This solution includes an oil in its formulation, differing from the invention proposed here.

Moreover, document FR 2 664 162 describes the cosmetic composition of a make-up designed to protect the skin from the harmful effects of air pollution, avoiding the excessive formation of free radicals that cause early aging of the skin. The composition basically contains a composition of babassu oil, dog-rose and honey glycolic extract, and may also contain pigments.

Document WO 2009/038712 presents a stable silicon gel composition containing boron nitride, which comprises powdered boron nitride, silicone oil and other cosmetically acceptable components. Once again, this is a solution quite different from that addressed by this invention.

Document PI 0414621-2 presents a composition for cleaning or caring for the skin, comprising a composition of solid particles of a single smooth flat crystal, with a refraction index of around 1.8 to around 2.2, selected from among bismuth oxychloride, boron nitride and others.

Document WO 2009/037347 addresses a non-aqueous composition that includes an oily phase and a powdery phase, with the latter phase involving 15% to 40% in mass of at least one pigment, fibers and at least one reticulated elastomeric organopolysiloxane in powdered form, and with the oily phase encompassing at least one hydrocarbon dispersant. This composition is intended to cover up irregularities of skin color, particularly under the eyes, as well as redness, scars and other marks.

Document EP 1 889 598 discloses an anhydrous cosmetic composition in a powder form, encompassing at least one powder phase and one grease-based binding agent, with this powder phase encompassing (i) 30% to 70% in mass of at least one pigment and (ii) 3% to 70% in mass of at least one lamellate particle.

However, any prior art document presents information on the use of babassu polysaccharide in cosmetic compositions intended for making up the skin, or even addresses the perceived and technical advantages achieved through the approach addressed by this invention, which will be presented below.

### BRIEF DESCRIPTION OF THE INVENTION

The objective of the invention is a cosmetic composition intended for making up the skin (face and body) that comprises:
(a) a sensorial modification system that encompasses at least babassu polysaccharide;
(b) at least one pigment; and
(c) at least one optical diffuser.

Another objective of this invention consists of cosmetic products with this composition, as well as the cosmetic use of babassu polysaccharide as a sensorial modifier.

### DETAILED DESCRIPTION OF THE INVENTION

This invention addresses a cosmetic composition intended particularly for making up the skin that comprises an exclusive combination of (a) a sensorial modification system encompassing at least babassu polysaccharide, (b) at least one pigment and (c) at least one optical diffuser.

The cosmetic composition addressed by this invention may be encompassed by cosmetic products such as powder foundation, pressed powders, eye shadows, blushers and other variations in powdered make-up for the skin.

Additionally, the cosmetic composition addressed by this invention presents a series of advantages and characteristics that are desirable in a cosmetic product for the skin, with advantages achieved through the optimum combination of the components already described, some of which are listed below:
- the cosmetic composition addressed by this invention makeup the skin evenly, with full coverage of the skin region where the composition was applied, good adhesion and minimization of excessive shine;
- the presence of optical diffuser and pigments, particularly treated pigments, provides the skin with a long-lasting natural-looking finish;
- the presence of babassu polysaccharide leaves the skin feeling soft, satiny and velvety;
- the sensorial modifier is plant-based (babassu polysaccharide), which is a desirable characteristic from the cosmetic standpoint;
- the cosmetic composition addressed by this invention is suitable for all skin types;
- the cosmetic composition addressed by this invention does not dry the skin and maintains its natural moisture;
- optionally, the cosmetic composition addressed by this invention may include protection against free radicals, through passionfruit ceramids encapsulated in cyclodextrin that leave the skin feeling softer, velvety and revitalized;
- the cosmetic composition addressed by this invention has been tested dermatologically and ophthalmologicaly and does not cause irritation;
- the cosmetic composition addressed by this invention does not present comedogenicity;
- the cosmetic composition addressed by this invention does not clog the pores, allowing the skin to breathe;
- the cosmetic composition addressed by this invention does not make the skin oilier, as it is already oil-free;
- in a preferred embodiment, it presents an ultrafine texture, achieving a homogeneous natural-looking;
- the cosmetic composition addressed by this invention is soft and slippery, offering comfort that responds to consumer wishes;
- it leaves the skin looking brighter and more radiant;
- it provides a soft focus skin effect (minimizing lines, wrinkles and the marks of time through a visual effect).

The main examples of cosmetic products that may be prepared on the basis of the cosmetic composition addressed by this invention are:
- Pressed face powder face;
- Loose face and body powder;
- Eye shadows;
- Blusher;
- Concealer for reddened areas, scars and patches;
- Foundation powder;
- Undercoat (make-up primer).

The cosmetic composition addressed by this invention has a combination of sensorial modifier, pigment and light diffusing particles that provided it with a unique feel and smoothness. Furthermore, in a preferred embodiment, it consists of a micronized product with an ultrafine texture and silky touch.

### SENSORIAL MODIFICATION SYSTEM

The sensorial modifier is used in quantities varying between 5% to 60% in mass, preferably 8% to 20% in mass, based on the mass of the composition. This sensorial modifier is an agent that absorbs skin oils, resulting in a soft, silky, delicate touch.

Examples of sensorial modification components that may be added are babassu polysaccharide (*Orbgnya phalerata*), polymethylacrylates, magnesium and aluminum silicates, polyacrylamides, polysaccharides, borates, silicas, nylon, talc, lauroyl lysine, micas in general and mica coated with lauroyl lysine.

In a preferred materialization, babassu polysaccharide is added to the sensorial modification system.

This babassu polysaccharide (*Orbgnya phalerata*) offers technical advantages compared to the other components; more particularly, it is preferable to add babassu polysaccharide extracted from the babassu found in Brazilian biodiversity, more specifically that obtained from the COOPAESP (Cooperativa dos Pequenos Productores Agroextrativistas de Esperantinópolis Ltda), an agricultural cooperative located in Esperantinópolis, Maranhão State.

In a particular embodiment, the babassu polysaccharides addressed by this invention present physical characteristics with unusual potential applications, namely:
1) Pink in color and rounded in shape with 90% granulometry of the particles between 60 and 80 µm; this raw material may be applied to the formulation of micronized pressed powder for make-up, replacing mineral talc. This new application presents excellent sensorial outcomes, compared with the formula including mineral talc.
2) Pink in color and rounded in shape with an average granulometry of 25µm; this raw material was assessed by a sensorial panel at concentrations of 2% and 4% presenting outcomes that surpass those of formulations using Nylon 12 (Polysensi - substitute Nylon 12).

Thus, the babassu polysaccharides present sensorial properties that could substitute Nylon 12, as an oil adsorbent in cosmetic formulas. As an additional benefit, an intermediate fraction of these polysaccharides is supplied as a new raw material with sensorial properties for substituting mineral talk in cosmetic formulations.

In an alternative embodiment, babassu polysaccharide blended with mica coated with lauroyl lysine or micas in general is added, in quantities varying between 8% and 20% in mass, but preferably varying between 10% and 15% in mass, with the quantities being based on the total mass of the composition.

### PIGMENT

The pigment is responsible for the coloring attained with the composition of the present invention, constituting an essential component for attaining the technical advantages already described.

The pigment used in the cosmetic composition of this invention is present in quantities of up to 40% in mass, preferably between / among 0.5 and 8% in mass.

This pigment encompasses treated or untreated pigments. Preferably, treated pigments are used.

Examples of untreated pigments include bismuth oxychloride (pearl), iron oxides, titanium dioxide, aluminum or barium or mica flakes or micas.

For bismuth oxychloride (pearl), this is a pearly pigment that endows the finished product with a spectacular effect, consisting of a velvety texture with powdered products adhering closely to the skin, excellent coverage, excellent compressibility and low oil absorption. It may also be used in products that need a pearly glow, with high transparency or opacity, as the pearly effect is highly appreciated, due to its lustrous properties and beauty.

Thanks to their shape, bismuth oxychloride crystals line up easily in parallel layers and, as they are transparent, each crystal reflects only part of the incident light. This simultaneous reflection of light along all the microscopic layers ensures a pearly luster.

Preferably, the UCR (Ultraviolet Completely Resistant) line is used, which ensures stability for UV radiation.

The untreated pigments have hydroxyl groups and are consequently potentially hydrophilic, absorbing water on the surface of the molecule, which may form clumping through forces attracting the hydrogen bonds.

Thus, the pigments may be treated in order to modify their physical, chemical and sensorial characteristics, in order to avoid clumping. The treatment technology results in a clearly perceptible sensorial improvement, with better application and wettability of the pigments, in addition to formula stability.

Preferably, the ITT (Isopropyl Titanium Triisostearate) treatment technology is used in the cosmetic composition addressed by this invention, which can provide even, lipophilic coverage with the stearic groups found in the molecule, being quite compatible with all the components in the formulation. This is very important in order to ensure a high final performance for the product.

The ITT coating is plant-based, ensuring close affinity with the esters used in the formulations with the pigments, resulting in greater stability for the formula and spreading more evenly. Furthermore, improvements to sensorial aspects and the fabrication process must also be stressed.

Consequently, this affinity also results in a long-lasting cosmetic composition, as the fine layer deposited on the skin can retain its color for longer periods of time, with high uniformity and ideal coverage. This is similar to a veil of color that blends into the micro-texture of the skin, resulting in a natural, long-lasting effect.

Furthermore, the treated pigment allows a high particle load to be attained for good coverage, helping in the optical diffusion / dissemination properties of the optical diffuser that will be described in detail below, with no negative alterations to its properties, in terms of application and fluidity. This must be due to the fact that pigment coated with ITT absorbs far less of the vehicle than a normal pigment (when the vehicle is present), meaning that the pigment is protected and stable, but the color and its dispersal are flawless and extremely fluid.

In a preferable embodiment, at least one treated pigment is added, preferably iron oxide treated with ITT in a quantity of up to 40% in mass, preferably between / among 0.5% and 8%, in mass, but preferably varying from 1% to 7.5% in mass, with the quantities being based on the total mass of the composition.

### OPTICAL DIFFUSER

The optical diffuser is a chemical component that diffuses, scatters or distributes light in some way, for more gentle illumination.

Examples of optical diffusers that may be used in this invention are boron nitride, silica, methyl methacrylate crosspolymer, nylon and a combination of mica, titanium dioxide and polymethylmethacrylate.

This optical diffuser is used in quantities varying between 3% and 30% in mass.

Preferably, boron nitride is used as an optical diffuser, with a special structure. Boron nitride is a crystal with a lamellate structure similar to graphite (hexagonal structure). This is an extremely white powder with high diffuse transmittance, in addition to lubricity (can be applied gently and easily), extended duration (adherence) and oil absorption (boron nitride absorbs sebum, allowing the formulation to remain on the skin longer). Due to its graphite structure, boron nitride particles have rounded ends, leaving it smoothly and silky.

In a preferable embodiment, boron nitride is added with a special structure as an optical diffuser, in quantities varying between 5% and 30% in mass, but preferably varying between 8% and 29.5% in mass, with the quantities being based on the total mass of the composition.

Thus, this preferable materialization results in ideal coverage that ensures uniformity with a natural-looking finish resulting from the use of high-technology treated pigments, finally disguising fine lines with a velvety finish through an optical diffuser (boron nitride).

### OPTIONAL COMPONENTS

In order to endow the cosmetic composition of the present invention with some desirable characteristic that has not yet been obtained with the components already mentioned above, optional components may be added that are compatible with the properties thereof. Some of these components that may be added to the composition are described below.

### BINDING AGENTS

Binding agents correspond to a blend of largely liquid components that provide a "binding" property for the cosmetic composition in powder form addressed by this invention.

Preferably, in order to obtain better homogenization, liquid binding agents are added to the cosmetic composition of the present invention through pulverization.

The concentration and quality of the binding agents must be assessed in order to ensure good adhesion for the particles while preserving cosmetic (result on the skin) and esthetic properties (pressed or loose) of the end product, which will be easily determined by a person skilled in the art. In other optional embodiments, binding agents are added in quantities varying between 4% and 10% in mass, with the quantities being based on the total mass of the composition.

Noteworthy among these properties is the property of removal of the cosmetic composition when in pressed form, through an applicator that is normally used for make-up, without the composition crumbling or breaking up when being handled.

The binding agents that are most recommended for adding to the cosmetic composition are magnesium stearate, octyldodecyl stearate, octyldodecyl stearoyl stearate and ethylhexyl palmitate.

When blended in equal proportions, octyldodecyl stearate and octyldodecyl stearoyl stearate can be pulverized easily, with excellent binding power.

When used in the cosmetic composition addressed by the present invention, ethylhexyl palmitate presents an excellent performance for use with very dry skins, with excellent spreading properties due to its velvety touch. It also presents an excellent performance through reducing the oily feeling of formulations that use oils and lipid components.

When used in sunscreens, it does not influence the active ingredient, in terms of the maximum ultraviolet wave length absorption (max). Furthermore, as this is a saturated ester, it is quite resistant to oxidation. It does not contain any materials that are animal in origin.

### VEHICLE

The vehicle is the constituent with the highest concentration in the cosmetic composition addressed by this invention. Preferably, the vehicle is a combination of babassu polysaccharide and sericite (mica).

Sericite is a mineral that is similar to mica in its chemical composition and shape, with extra-fine particle size and a silky matt finish. Sericite is softer and gentler than conventional mica, with a touch comparable to an oil, and excellent spreadability. Available in lamellate format. When compared to mica, sericite contains more water for moisturizing in its structure, ensuring superior softness and smoothness.

The vehicle is added to the cosmetic composition of the present invention in quantities varying between 40% to 75%, more particularly 45% to 60% in mass, with the quantities being based on the total mass of the composition.

### ADDITIONAL ACTIVE INGREDIENT

If desired, additional active ingredients can be added to the cosmetic composition of this invention.

In another embodiment, passionfruit ceramids encapsulated in β-cyclodextrin may be added, for revitalizing the skin.

β-cyclodextrin has a hydrophilic character, meaning that the complex blended with the passionfruit ceramids tends to present lower cutaneous permeation, remaining on the skin surface.

Due to the dynamic nature of this molecular encapsulation, it may be inferred that the cyclodextrin gradually releases the passionfruit ceramids, thus extending the restorative effect of the cutaneous barrier provided by this active ingredient.

The forms of the oils and waxes included in β-cyclodextrin are altered to a fine powder, thus allowing it to be blended into powdered cosmetic formulations, allowing a wider diversity of active ingredients to be used in a line of make-up, with skin treatment benefits.

Other active ingredients may also be added to the cosmetic composition addressed by this invention, such as an anti-aging ingredient, for example.

In a preferable embodiment, at least one active ingredient is added in quantities varying between 2.5% and to 5% in mass, preferably between 2.5% and 3% in mass, with the quantities being based on the total mass of the composition.

### ANTI-OXIDANT COMPLEX

Anti-oxidants can protect the skin against free radicals, which are highly reactive molecules that can interact and destroy various types of important structures in the skin.

In a preferred embodiment of the present invention, at least one anti-oxidant is added in quantities varying between 0.002% and 0.2%, in mass, with the quantities being based on the total mass of the composition.

In a preferable embodiment, the anti-oxidant complex developed by the Applicant is added, consisting of a blend of coffee extract, lycopene and vitamin E. This ant-oxidant is described in brief below:

Coffee Extract - the chemical composition of coffee contains 1% to 2.5% of caffeine and assorted other substances that are present in higher concentrations, such as chlorogenic acids for example. Chlorogenic acids possess high anti-oxidant capacities and are found in green coffee in concentrations that are three to five times higher than caffeine.

Through an elaborate extraction process, the chlorogenic acids present in the coffee are extracted and concentrated until obtaining a highly purified extract with excellent anti-oxidant capacities for the skin.

On the skin, the anti-radical action of the coffee extract has been proven through specific studies demonstrating its ability to reduce the oxidation of lipids (fats) of the skin cell membranes. Thus, coffee extract helps preserve important cell architecture structures.

Lycopene - this is a substance belonging to the chemical group of the carotenoids, which are natural pigments synthesized by plants and some micro-organisms. Belonging to the same family as beta-carotene, it is extracted from tomatoes, endowing them with their red color. Many studies have demonstrated its properties, due to its special structure, with lycopene rated as the carotenoid with the highest potential for combating free radicals, mostly against one of the most important types of free radicals generated by the body (there are different types of free radicals that act on our bodies).

Vitamin E - plays a key role in preserving the cell membranes through capturing free radicals formed during biochemical reactions of the skin or triggered by exposure to the sun. Thus, Vitamin E interrupts the cascade effect that forms more free radicals, protecting skin structures (colloidal gel, elastin and collagen), while minimizing their degradation.

Studies have shown that the anti-oxidant complex encompassing lycopene, vitamin E acetate and coffee extract presents synergic effects, thus helping the body's natural defense system combat free radicals more effectively, when formed through external aggressive factors such as ultraviolet rays.

### UVA AND UVB SUNSCREENS

The sun is the main factor causing signs of aging in the skin, with UVB radiation prompting the appearance of erythema, sunburn and patchiness, while UVA radiation causes the destruction of support fibers (collagen and elastin), leaving the skin flabby and wrinkled.

Consequently, broad spectrum sunscreen is crucial for preventing photo-aging (UVA and UVB). In a preferred embodiments, a sunscreen system is added to the cosmetic composition addressed by the present invention, with photo-stable physical protection (titanium dioxide and zinc oxide), that endow it with FPS 15 (UVB shield) and also protect against UVA rays, ensuring efficiency and safety, while also providing a pleasant feeling during and after the application.

In embodiments of the invention, at least one sunscreen is added, in quantities varying between 10% and 20% in mass, with the quantities being based on the total mass of the composition.

### EXAMPLES

The compositions described above were tested, with the outcomes of the tests set forth in detail below.

### Example 1

### Potential Cutaneous Irritability, Sensitization, Photo-Allergy and Photo-Toxicity (Patch Test) - Pressed Powder

Heightened awareness in the industry and consumer demands have prompted the adoption of a new procedure by cosmetic manufacturers: today, companies are concerned about conducting clinical trials coordinated by dermatologists, thus ensuring the safety and efficacy of their products before presenting them for sale. This procedure endows the company with greater safety, credibility and trust among its consumers.

A matter of rising concern in the cosmetics industry is to avoid possible adverse reactions among the users of its products. After all, these consumers are far more critical when skin is irritated by a cosmetic product than by a topical medication.

The patch test is the main tool used for diagnosing a reaction caused by a cosmetic, as well as in allergenicity trials. As the main potential risks arising from the use of a new product are irritation, sensitization allergy, photo-toxicity and photo-allergy, the allergenicity trials include the following clinical tests: primary and accumulated dermal irritability, skin sensitization, photo-toxicity and photo-allergy. These consist of repeated applications of the product to the skin, in order to detect possible irritations or heightened sensitization.

In addition to allergenicity, these tests can also assess the sensorial characteristics of the product, detecting complaints and additional comments on its performance.

Results: according to the methodology used to assess the potential for causing irritability, sensitization, photo-allergy and photo-toxicity of the product described in Example 1, it may be concluded that the product does not cause any cutaneous irritation or sensitization, and did not trigger photo-allergy and photo-toxicity during the trial period.

### Controlled Dermatological and Ophthalmological Use Test

The purpose of this study was to ascertain the cutaneous and ocular acceptability of the composition as a pressed powder, under normal conditions of use. The skin and eye acceptability outcomes were:
- Checked daily by the volunteers in their homes.
- Controlled after visual examinations of the experimental area by the Investigator or Researcher/Technician, under the responsibility thereof, and after a questionnaire was completed by the volunteers, in addition to a visual inspection with a slit lamp conducted by an ophthalmologist.

The experimental conditions adopted (experimental area, quantities of product applied, frequency and duration of the applications, etc.) reproduced normal conditions of use, and this small-scale test reflected the application as used by future consumers.

The cutaneous acceptability of the product was controlled by a dermatologist with appropriate experience or an experienced and well qualified researcher/technician under the responsibility thereof. The ocular acceptability of the product was assessed by an ophthalmologist using a slit lamp.

As this involves a confirmation of cutaneous and ocular acceptability and as the applications and examinations are tightly controlled, the number of volunteers defined in the protocol is sufficient to assess possible irritations.

Results: the cutaneous and ocular acceptability results were expressed mainly as descriptive data, with no statistical treatment required. According to the test conditions, the results obtained in the manner of use and the rating scale indicated by the manufacturer lead to the conclusion that the pressed powder mode presented no reactive volunteers.

### Comedogenicity

Selected volunteers were assessed clinically by a dermatologist at the start of this study (T0). Next, a qualified practitioner counted the number of comedos (blackheads) in an area that was pre-determined by a template with the width of 4.0 cm by 4.5 cm long, on part of the face, with the assistance of magnifying light appropriate for this purpose, as shown in the following drawing.

Forehead Area: area with a width of 4.0 cm and a length of 4.5 cm, located evenly between the mid-point of the glabella and the mid-point of the forehead line through an imaginary line.

Right or Left Half Face Area: area with a width of 4.0 cm and a length of 4.5 cm, located evenly between the mid-point of the lower eyelid and the mid-point of the mentum line through an imaginary line.

After assessment, the samples were handed over to the volunteers as described in the guidelines related to the manner of use, demonstrated below.

After 30 days of use, the volunteers returned for dermatological assessment (T30), in order for the number of comedos (blackheads) to be counted by a trained beautician. All the described procedures were noted on the clinical datasheet and the comedo counting leaflets of the volunteers.

Results: for the conditions under which the product was assessed and the sample of volunteers studied, the data leads to the conclusion that no comedogenic potential was noted under the conditions of use during the assessment period.

### Example 2

### Potential for Cutaneous Irritability, Sensitization, Photo-Allergy and Photo-Toxicity (Patch Test) - FPS 15 Foundation Powder

The protocol for this safety test conducted for this composition is equivalent to that described for Example 1.

Results: according to the methodology used to assess the potential form cutaneous irritability, sensitization, photo-allergy and photo-toxicity of the product illustrated in Example 2, it may be concluded that the product did not cause any cutaneous irritation and sensitization, and did not cause photo-allergy and photo-toxicity during the study period.

### Usage Test Under Dermatological and Ophthalmological Controls

The purpose of the study was to ascertain the cutaneous and ocular acceptability of the FPS 15 foundation powder under normal conditions of use.

The protocol for this test conducted for the composition given in Example 2 is equivalent to that described for Example 1.

Results: according to the test conditions, the results, the manner of use and the rating scale indicated by the manufacturer, leads to the conclusion that the composition of Example 2 did not present any reactive volunteers.

### Comedogenicity

The protocol for this test conducted for this composition is equivalent to that described for Example 1.

Results: under the conditions in which the product was assessed and in the sample of volunteers studied, the data lead to the conclusion that no comedogenic potential was observed under the conditions of use, during the assessment period.

### Example 3

### Potential for Cutaneous Irritability, Sensitization, Photo-Allergy and Photo-Toxicity (Patch Test) - Pressed Eye Shadow

The following tests were conducted for the pressed eye shadow mode:

The protocol for the safety test conducted for this composition is equivalent to that described for Example 1.

Results: according to the methodology used to assess the potential for cutaneous irritability, sensitization, photo-allergy and photo-toxicity of the product illustrated in Example 3, it may be concluded that the product did not cause any cutaneous irritation and sensitization, and did not result in any photo-allergy and photo-toxicity during the study period.

### Usage Test Under Dermatological and Ophthalmological Controls

The purpose of this study was to ascertain the cutaneous and ocular acceptability of the pressed eye shadow under normal conditions of use.

The protocol for the test conducted for this composition is equivalent to that described for Example 1.

Results: according to the test conditions, results, manner of use and rating scale indicated by the manufacturer, it was concluded that the composition addressed in Example 3 did not present any reactive volunteers.

### Comedogenicity

The protocol for the test conducted for this composition is equivalent to that described for Example 1.

Results: under the conditions in which the product was assessed and in the sample of volunteers studied, the data lead to the conclusion that no comedogenic potential was observed under the conditions of use, during the assessment period.

### Example 4

### Potential for Cutaneous Irritability, Sensitization, Photo-Allergy and Photo-Toxicity (Patch Test) - Blusher

The following tests were conducted for the Blusher mode:

The protocol for the safety test conducted for this composition is equivalent to that described for Example 1.

Results: according to the methodology used to assess the potential for cutaneous irritability, sensitization, photo-allergy and photo-toxicity of the product illustrated in Example 4, it may be concluded that the product did not cause any cutaneous irritation and sensitization, and did not result in any photo-allergy and photo-toxicity during the study period.

### Usage Test Under Dermatological and Ophthalmological Controls

The purpose of this study was to ascertain the cutaneous and ocular acceptability of the Blusher, under normal conditions of use.

The protocol for the test conducted for this composition is equivalent to that described for Example 1.

Results: according to the test conditions, results, manner of use and rating scale indicated by the manufacturer, it was concluded that the composition addressed in Example 4 did not present any reactive volunteers.

### Comedogenicity

The protocol for the test conducted for this composition is equivalent to that described for Example 1.

Results: under the conditions in which the product was assessed and in the sample of volunteers studied, the data lead to the conclusion that no comedogenic potential was observed under the conditions of use, during the assessment period.

## Claims

1. Cosmetic composition intended for making up the skin **characterized in that** comprises:
- one sensorial modification system that encompasses at least a babassu polysaccharide;
- at least one optical diffuser;
- at least one pigment.

2. Composition according to claim 1, **characterized in that** the sensorial modifier is used in quantities varying between 5% to 60% in mass.

3. Composition according to claim 2, **characterized in that** the sensorial modifier is used in quantities varying between 8% to 20% in mass.

4. Composition according to any of claims 1 to 3, **characterized in that** the sensorial modification system further comprises one sensorial modifier selected from the group consisting of polymethylacrylates, magnesium and aluminum silicates, polyacrylamides, polysaccharides, borates, silicas, nylon, talc, lauroyl lysine, micas in general and mica coated with lauroyl lysine.

5. Composition according to claim 1, **characterized in that** the sensorial modification system comprises pink babassu polysaccharide, rounded in shape with 90% granulometry of the particles between 60 and 80 µm.

6. Composition according to claim 1, **characterized in that** the sensorial modification system comprises pink babassu polysaccharide, rounded in shape with an average granulometry of 25µm.

7. Composition according to claim 1, **characterized in that** the sensorial modification system comprises babassu polysaccharide blended with mica or mica coated with lauroyl lysine in quantities varying between 8% and 20% in mass.

8. Composition according to claim 7, **characterized in that** the babassu polysaccharide blended with mica or mica coated with lauroyl lysine is present in a range of 10% to 15% in mass.

9. Composition according to any of Claims 1 to 8, **characterized in that** it comprises pigments in a quantity of up to 40% in mass.

10. Composition according to claim 9, **characterized in that** it comprises pigments in a quantity of 0.5 and 8% in mass.

11. Composition according to claims 9 or 10, **characterized in that** it comprises treated or untreated pigments.

12. Composition according to claim 11, **characterized in that** it of untreated pigments are selected from the group containing bismuth oxychloride (pearl), iron oxides, titanium dioxide, aluminum or barium or mica flakes.

13. Composition according to any of claims 9 to 11, **characterized in that** it comprises treated pigments.

14. Composition according to claim 13, **characterized in that** the treated pigment is present in a quantity of up to 40% in mass, based on the total mass of the composition.

15. Composition according to claim 13 or 14, **characterized in that** the treated pigments are selected from among bismuth oxychloride, iron oxides, titanium dioxide, aluminum or barium or mica flakes or treated micas coated with ITT (isopropyl titanium triisostearate).

16. Composition according to any of claims 13 to 15, **characterized in that** the treated pigment is iron oxide treated with ITT.

17. Composition according to claim 16, **characterized in that** the iron oxide treated with ITT is present in a quantity of up to 40% in mass.

18. Composition according to claim 17, **characterized in that** the iron oxide treated with ITT is present in a quantity between 0.5% and 8% in mass.

19. Composition according to claim 18, **characterized in that** the iron oxide treated with ITT is present in a quantity between 1% and 7.5% in mass.

20. Composition according to any of claims 1 to 20, **characterized in that** the optical diffuser is present in quantities varying between 3% and 30% in mass, based on the total mass of the composition.

21. Composition according to any of claims 1 to 21, **characterized in that** the optical diffuser is selected from the group consisting of boron nitride, silica, methyl methacrylate crosspolymer, nylon and a combination of mica, titanium dioxide and polymethylmethacrylate.

22. Composition according to claim 21, **characterized in that** the optical diffuser is boron nitride.

23. Composition according to any of claims 21 and 22, **characterized in that** the optical diffuser is boron nitride with a structure containing particles with rounded ends.

24. Composition according to any of claims 20 to 23, **characterized in that** the optical diffuser is boron nitride in quantities varying between 5% and 30% in mass.

25. Composition according to any of claims 20 to 24, **characterized in that** the optical diffuser is boron nitride in quantities varying between 8% and 29.5% in mass.

26. Composition according to any of claims 1 to 25, **characterized in that** it comprises at least one binding agent selected from among magnesium stearate, octyldodecyl stearate, octyldodecyl stearoyl stearate, ethylhexyl palmitate and blends thereof.

27. Composition according to claim 26, **characterized in that** the binding agent is present in a quantity of 4% to 10% in mass.

28. Composition according to any of claims 1 to 27, **characterized in that** it comprises at least one vehicle, being a combination of babassu polysaccharide and sericite (mica).

29. Composition according to any of claims 1 to 28, **characterized in that** it comprises additional active ingredients.

30. Composition according to claim 29, **characterized in that** the additional active ingredients are passionfruit ceramids encapsulated in β-cyclodextrin or anti-aging agents.

31. Composition according to claim 29 or 30, **characterized in that** the additional active ingredients are present in quantities varying between 2.5% to 5% in mass, based on the total mass of the composition.

32. Cosmetic composition according to claim 1 **characterized in that** it optionally comprises an anti-oxidant complex that consists of a combination of coffee extract, lycopene and vitamin E.

33. Composition according to claim 32, **characterized in that** the anti-oxidant complex is present in quantities varying between 0.002 % and 0.2% in mass, based on the total mass of the composition.

34. Composition according to claim 1, **characterized in that** the anti-oxidant complex is present in quantities varying between 10% and 20% in mass, based on the total mass of the composition.

35. Cosmetic composition according to claim 1, **characterized in that** it optionally comprises a sunscreen system with protection against UVA and UVB rays with physical screens, such as titanium dioxide and zinc oxide.

36. Cosmetic product, **characterized in that** it comprises a cosmetic composition as defined in any of Claims 1 to 35.

37. Product according to claim 36, **characterized in that** it is a pressed powder.

38. Product according to claim 36, **characterized in that** it is a loose powder.

39. Product according to claim 36, **characterized in that** it is a foundation powder.

40. Product according to claim 36, **characterized in that** it is a pressed eye shadow.

41. Product according to claim 36, **characterized in that** it is a blusher.

42. Product according to claim 36, **characterized in that** it is a concealer.

43. Product according to claim 36, **characterized in that** it is an undercoat (make-up primer).

44. Use of babassu polysaccharide, **characterized in that** it is used as a one sensorial modifier in a cosmetic composition intended for making up the skin.
